# FASCICULE DE BREVET EUROPEEN

(11) **EP 3 547 953 B1**
(45) Date de publication et mention de la délivrance du brevet: **21.04.2021**
(21) Numéro de dépôt: 16815621.4
(22) Date de dépôt: 30.11.2016
(51) Int. Cl.: A61C 8/00

(54) **IMPLANT DENTAIRE ET ELEMENT DE FIXATION AUTOBLOQUANT A STRUCTURES POREUSES HETEROGENES ET SON PROCEDE DE FABRICATION**
ZAHNIMPLANTAT UND SELBSTSPERRENDES BEFESTIGUNGSELEMENT MIT HETEROGENEN PORÖSEN STRUKTUREN UND VERFAHREN ZU DESSEN HERSTELLUNG
DENTAL IMPLANT AND SELF-LOCKING FASTENING ELEMENT WITH HETEROGENEOUS POROUS STRUCTURES, AND METHOD FOR ITS PRODUCTION

(43) Date de publication de la demande: 09.10.2019
(73) Titulaire: Djemai, Abdelmadjid, 95170 Deuil La Barre (FR)
(72) Inventeur: FOUCHET, Jean-Jacques, 45700 Lombreuil (FR); DJEMAI, Abdelmadjid, 45700 Lombreuil (FR)
(74) Mandataire: IP Trust
(86) Numéro de dépôt international: PCT/FR2016/053154
(87) Numéro de publication internationale: WO 2018/100250

(56) Documents cités:
- EP-A1- 2 836 168
- WO-A1-01/05325
- US-A1- 2010 003 639
- US-A1- 2011 111 368

## Description

### DOMAINE TECHNIQUE

L'invention concerne un ensemble formant un implant dentaire comportant un corps interne de forme allongé ressemblant sensiblement à une racine de la dent, et un élément de fixation à l'os d'un patient, comprenant une structure poreuse hétérogène assurant la croissance osseuse.

L'implant est l'infrastructure généralement métallique destinée à soutenir une prothèse dentaire. Il permet de remplacer les piliers naturels que sont les dents, par des piliers mécaniques, placés soit dans l'os mandibulaire, soit dans l'os maxillaire.

L'essentiel dans la pose d'un implant dentaire est d'obtenir un blocage immédiat dans l'os. Une mobilité même faible de l'implant dans l'os entraîne à terme un rejet, une faible disparité entre l'implant et l'os peut entrainer une résorption osseuse.

### ETAT DE LA TECHNIQUE ET SES INCONVENIENTS

Les techniques actuelles de fabrication d'implants dentaire sont des techniques traditionnelles d'usinage, en général d'une tige filetée. L'implant ainsi réalisé a une surface lisse et brillante. Une fois l'implant introduit dans l'os, une bague taraudée ou vis est mis en place sur la partie extérieure qui est en général une tige filetée. Sur ladite bague taraudée ou vis, est fixée par un ciment la prothèse dentaire.

Les principales causes du rejet des implants dentaires sont la mobilité de l'implant (même faible) et la résorption osseuse due en général à l'état de surface de l'implant.

L'état de la technique peut être défini par deux catégories de brevets :
1) des brevets d'implants réalisés par usinage, citons le brevet de Jean-Marc JUILLET déposé le 12 juin 1972 sous le No. 72 21113 et publié le 2 janvier 1974 sous le No. 2.188.445,
2) d'autres brevets qui traitent des traitements de surface pour l'oséo intégration, nous citons le brevet de Zimmer Dental « Dental implant with improved osseointegration features » dépose le 30 août 2006 sous le numéro US807512 B2.
   Le brevet de Conformis Inc. « Devices and methods for additive manufacturing of implants components" déposé le 13 avril 2013 sous le numéro WO 2013155500 A1
   Le brevet de Eric Jones « Laser produced porous surfaces » dépose le 6 décembre 2005 sous le numéro US20070142914.
   Le brevet de Howmedica Osteonics Corp. « Laser produced implants » dépose le 29 décembre 2006 sous le numéro US20080004709
   Tous ces brevets décrivent des moyens et méthodes de traitement de surface ou une technique de dépôt d'endospores de Tantale par la méthode de dépôt chimique sous forme vapeur, la porosité obtenue en en moyenne de 35% et l'interconnectivité partielle.
3) les brevets d'implants réalisés par fabrication additive (impression 3D), nous citerons le brevet de l'université de Liverpool déposé le 9 juin 2010 sous le numéro WO2010146383A1.

Ce brevet décrit un implant dentaire réalisé par fabrication additive, où un procédé de fabrication par fusion sélective laser est longuement expliqué, mais il n'apporte aucune solution aux problèmes du blocage de l'implant à la mise en bouche et n'apporte ainsi aucune solution afin d'éviter un rejet de l'implant.

Parmi les nombreuses publications traitant du sujet de la fabrication additive d'implants dentaire, les suivantes s'accordent sur les avantages indéniables de la réalisation d'implants dentaires par fabrication additive dans des matériaux bio compatibles de type Ti6Al4V dans différents grades :
« Direct Metal Laser Sintering Titanium Dental Implants : A Review of the current Literature » publiée le 01 décembre 2014 dans International Journal of Biomaterials.

« Histomorphometric Evaluation of Direct Laser Metal Forming (DLMF) Implant Surface in the Type IV Bone: A Controlled Study in Human Jaw" Publié le 25 juillet 2013 dans la revue POSEIDON Journal « Fabrication of Bioactive Porous Ti Metal with Structure Similar to human Cancellous Bone by Selective Laser Melting" Publié le 2 décembre 2010 dans la revue BDA Bioceramics Development and Applications.

La fabrication additive et pour certaines technologies additives précisément (SLM, EBM, SLA) présente plusieurs possibilités de contrôle de la géométrie, de la porosité, de l'inter connectivité et l'architecture 3D par des changements dans les paramètres de fabrication, citons les principaux paramètres pour la technologie de fusion laser sélective:
- la puissance des lasers
- la vitesse de scan
- le diamètre du spot
- la stratégie de parcours du laser
- le recouvrement entre deux point de fusion
- l'épaisseur des couches de la poudre

Le procédé de fusion laser sélective de poudre métallique en terminologie anglaise « Selective Laser Melting (SLM) », la dénomination SLM sera maintenue dans tout le texte du brevet.

SLM est un procédé utilisé pour la fabrication de composants complexes en trois dimensions à partir de poudres de métal, de céramique ou poudres polymère. La technologie est mature et déjà utilisée dans l'industrie aéronautique et le médical pour fabriquer des composants complexes présentant de hautes densités et homogénéité. Nous citons un des premiers brevets de l'institut Fraunhofer en Allemagne, déposé le 27 oct. 1997 sous le numéro WO1998024574A1, qui décrit le procédé SLM d'une façon plus précise

US 2011/111368 A1 divulgue un ensemble formant un implant dentaire selon le préambule de la revendication 1.

### EXPOSE DE L'INVENTION

L'invention remédie aux problèmes de l'art antérieur en proposant un ensemble selon la revendication 1. La présente invention concerne aussi un procédé selon la revendication 16.

A cet effet, l'invention concerne un ensemble formant un implant dentaire comprenant un corps interne de forme allongée ressemblant sensiblement à une racine de dent et un élément externe de fixation d'un os d'un utilisateur présentant une structure poreuse hétérogène apte à assurer la croissance osseuse, le corps interne et l'élément externe de fixation étant formés d'une seule pièce, l'élément de fixation étant mobile entre une position passive rangée et une position active déployée.

La constitution en une seule pièce de cet ensemble est en outre obtenu grâce à un procédé de fabrication additive de fusion de couches de poudre, notamment le procédé SLM précité.

L'ensemble selon l'invention peut par ailleurs présenter l'une et/ou l'autre des caractéristiques suivantes :
- le corps interne de l'implant comprend un filetage externe présentant une hauteur de filetage H, l'élément de fixation est interposé entre les spires du filetage et se déployant entre la position passive rangée et la position active déployée d'une distance égale ou supérieure à la hauteur de filetage.
- L'élément de fixation présente un coefficient de dilatation thermique supérieur à celui du corps de l'implant lui permettant un déploiement latéral supérieur de 15 à 40% vis à vis de celui du corps interne à température corporelle
- Le matériau constitutif de l'élément de fixation présente un coefficient de dilatation thermique supérieur à celui du corps de l'implant lui permettant un déploiement latéral supérieur de 1 à 20% à température corporelle
- l'ensemble selon l'invention comprend un dispositif amovible d'écartement de l'élément de fixation qui est inséré à l'intérieur du corps interne et qui commande le déplacement de l'élément de fixation de la position passive rangée à la position active déployée
- le dispositif amovible d'écartement est une vis de mise en place de l'implant
- l'élément de fixation est relié au corps interne par une liaison sécable
- l'élément de fixation et le corps interne ne présentent aucune liaison
- l'élément de fixation et/ou le corps interne sont constitués d'un matériau composite formé d'une fusion d'un mélange de microparticules d'un premier matériau à température de fusion donné, et de nanoparticules d'un deuxième matériau à température de fusion plus élevé, définissant un réseau de zones dendritiques du premier matériau à l'échelle du micron entremêlé de zones filamenteuses nanométriques du deuxième matériau.
- le premier matériau est un métal
- le deuxième matériau est une céramique
- le premier matériau comprend ou est du titane
- le deuxième matériau comprend ou est une zircone
- le deuxième matériau comprend ou est une zircone yttriée
- le corps interne présente une forme tronconique creuse définissant une épaisseur de paroi, pourvu d'un filetage externe, et comprend deux gorges opposées réalisées dans son épaisseur de paroi et suivant un profil hélicoïdal, l'élément de fixation présentant la forme d'un ruban épais torsadé selon le profil hélicoïdal des deux gorges et d'une forme complémentaire à celles ci
- l'élément de fixation comprend un évidemment longitudinal central de passage d'un dispositif d'écartement.

L'invention concerne également le procédé de fabrication d'un ensemble tel que décrit ci-dessus, par empilement de couches de poudres métalliques et/ou non métalliques, fusionnées sélectivement par concentration d'une source d'énergie.

Selon l'invention, ce procédé comprend une étape de préparation de la mobilité de l'élément de fixation par rapport au corps interne.

Le procédé selon l'invention peut par ailleurs présenter l'un et/ou l'autre des aspects suivants :
- l'étape de préparation de la mobilité de l'élément de fixation comprend une étape de formation d'un pont de matière sécable entre une partie constitutive de l'ensemble formant le corps interne et une partie constitutive de l'ensemble formant l'élément de fixation
- l'étape de préparation de la mobilité de l'élément de fixation comprend une étape de constitution de la partie de l'ensemble formant l'élément de fixation avec un matériau présentant un coefficient de dilatation thermique supérieur à celui du corps de l'implant lui permettant un déploiement latéral supérieur de 1 à 20% vis à vis de celui du corps interne à température corporelle
- l'étape de préparation de la mobilité de l'élément de fixation comprend une étape de formation de la partie de l'ensemble formant le corps interne et de la partie de l'ensemble formant l'élément de fixation sans pont de matière entre elles, la partie de l'ensemble formant l'élément de fixation étant fabriquée avec un évidemment interne lui permettant d'être déployable radialement sous l'effet d'une dilatation thermique et/ou d'un outil, indépendamment de la partie de l'ensemble formant le corps interne.

### DESCRIPTION DETAILLEE DES FIGURES

L'invention sera maintenant décrite plus en détail à l'aide des dessins qui illustrent des formes de réalisation préférentielles d'un ensemble suivant l'invention. Sur ces dessins :
- La FIGURE. 1 montre l'implant dentaire et l'élément de fixation selon un premier exemple de réalisation
- La FIGURE. 2 Illustre les différentes étapes du flux numérique du mode de réalisation de l'ensemble implant dentaire et élément de fixation selon la figure 1
- La FIGURE. 3 illustre un premier mode de réalisation de la cellule unitaire et sa mise en œuvre dans l'élément de fixation de la figure 1
- La FIGURE. 4 Illustre la mise en bouche de l'ensemble implant dentaire et élément de fixation (à gauche) et son auto blocage sur la paroi implantaire (à droite).
- La FIGURE 5 illustre un deuxième mode de réalisation de l'ensemble à implant dentaire et élément de fixation selon l'invention
- La FIGURE 6 représente une vue en coupe transversale et longitudinale d'un IRM réalisé sur un utilisateur portant l'implant de la figure 5 après 8 semaines de croissance osseuse (coupe transversale et coupe longitudinale, l'os étant représenté en foncé et l'implant en clair)

La présente invention décrit un système et son procédé par fabrication additive d'implants dentaire à structures poreuses hétérogènes et autobloquants qui apporte avantageusement une double réponse aux problèmes inhérents à l'implantologie dentaire à savoir l'oséo-intégration et la mobilité de l'implant lors de sa pose. Pour cela la présente invention propose un implant dentaire autobloquant à structures poreuses hétérogènes.

L'implant dentaire décrit dans la présente invention est composé d'un corps central qui représente la racine ancrée dans l'os de la mâchoire, la tête de l'implant peut être arrondie, plate, creuse, ou de tout forme particulière pour recevoir le pilier prothétique. Dans un autre mode de réalisation l'implant peut être solidaire du pilier implantaire. La réalisation en une seule pièce de l'implant et du pilier prothétique se fera par fabrication additive, des angles de courbure particuliers entre l'implant et le pilier sont calculés suite à un traitement numérique des images tridimensionnelles de la morphologie de la mâchoire.

Il est autobloquant dans l'os dans lequel il est implanté grâce à l'élément de fixation osseuse poreux, qui est rendu mobile entre une position rangée passive permettant une insertion de l'implant dans une perforation réalisée dans l'os, et une position déployée active où cet élément poreux comble les cavités existant entre le corps interne de l'implant et l'os et sera colonisé par cet os lors de la croissance osseuse.

Cette mobilité de l'élément de fixation peut être provoquée par une dilatation thermique, et/ou par un outil tel que cela sera explicité plus en détail dans ce qui suit.

### Elément de fixation poreux

L'élément de fixation peut présenter la forme d'un revêtement à structures poreuses hétérogènes de forme hélicoïdale pour certains implants à filetage mais ce revêtement peut être de différentes formes et géométries qui viennent en habillage d'une partie ou la totalité de l'implant dentaire en étant disposé de préférence dans la partie de l'implant dentaire au contact avec l'os.

Ladite structure présente une porosité comprise entre 30% et 80%, une taille des pores comprise entre 100 µm et 500 µm et une distribution des pores comprise entre 500 µm et 700 µm avec une inter connectivité totale. Dans un mode préférentiel la porosité comprise entre 60% et 70 %, la taille des pores comprise entre 200 µm et 300 µm avec une distribution de la porosité comprise entre 100 µm et 600 µm et une inter connectivité totale. Ses paramètres sont ainsi proches des caractéristiques de l'os humain dans la maxillaire et la mandibule.

### Corps interne de l'implant

Le corps de l'implant dentaire et son élément de fixation sont réalisés par empilement de couches de poudre métallique ou non métallique, fusionnés sélectivement. Dans le cas de poudre métallique d'alliage de titane/aluminium/vanadium, l'épaisseur des couches est généralement de 30µm. Une des techniques de fabrication additive sélectionnée pour notre exemple est la technologie SLM, ce mode de réalisation n'est pas limitatif, d'autres technologies de dépôt peuvent être déployé.

### Réalisation par addition

Un fichier numérique de l'implant dentaire est réalisé par un logiciel de conception en trois dimensions soit selon des modèles et normes standards ou soit selon un mode de réalisation particulier: dans ce cas, la forme de l'implant dentaire à savoir sa hauteur, sa section basse, sa section haute sont déterminés de manière plus précise.

Dans un autre mode de réalisation, l'implant dentaire peut avoir la forme exacte de la dent, ce mode est privilégié dans le cas de remplacement d'une dent juste après son extraction. On réalise ainsi un implant« sur mesure » en fonction de la morphologie et des propriétés mécaniques de la dent du patient à remplacer et/ou de l'os dans lequel l'implant sera intégré.

Dans ce fichier, l'élément de fixation est prédéfini avec une épaisseur particulière comprise entre 1 et 2 mm, dans un mode préférentiel l'épaisseur est comprise entre 0.8 mm et 1.5 mm.

Plusieurs formes de porosités peuvent être sélectionnées avec la possibilité de définir des zones de répartition des pores de différentes tailles.

Idéalement, la structure poreuse formant l'élément de fixation présente une porosité comprise entre 30% et 80%, une taille des pores comprise entre 100 µm et 500 µm et une distribution des pores comprise entre 500 et 700 µm avec une inter connectivité totale.

Dans un mode préférentiel la structure poreuse présente une porosité comprise entre 60% et 70 %, la taille des pores comprise entre 200 µm et 300 µm avec une distribution de la porosité comprise entre 100 et 600 µm et une inter connectivité totale.

La cellule de base ou cellule unitaire constitutive de la structure poreuse réalisée par empilement de couches, est de forme géométrique en trois dimensions (x,y,z), la cellule unitaire est formée par au moins trois arêtes avec une ouverture d'angle d'au moins 10°, telle un trièdre, et la cellule unitaire peut être de forme régulière ou irrégulier en forme de pyramide, tétraèdre, cubique, octaèdre, icosaèdre, dodécaèdre et sans limitation de forme. Dans un mode de réalisation préférentiel, la cellule unitaire sera de forme dodécaèdre renforcée.

Dans un autre mode de réalisation, la cellule unitaire est formée de 12 arêtes avec des angles d'ouverture des arêtes de 30° par rapport à l'axe vertical ou l'axe de construction. Les arêtes peuvent être régulières ou irrégulières selon la densité du maillage et la porosité voulue.

Le matériau utilisé pour la réalisation d'un tel implant est un matériau biocompatible de métal pur ou d'alliages métalliques de type Chrome cobalt, tantale, niobium, des composés métallo céramique ou organo-metal ou organo céramique ou une combinaison organo céramique métal.

Pour la réalisation du corps interne, deux alliages de matériaux ont été préférentiellement utilisés :
- un alliage Titane Aluminium vanadium Ti6Al4V de grade 23, avec un taux d'oxygène < 0.2 %,
- Une combinaison d'un alliage de titane mélangé avantageusement avec un matériau à base de zircone.

Pour la réalisation de l'élément de fixation, trois alliages ont été utilisés :
- Un alliage Titane Aluminium vanadium Ti6Al4V de grade 23, avec un taux d'oxygène < 0.2 %,
- Une combinaison d'un alliage de titane mélangé avantageusement avec un matériau à base de zircone
- Une combinaison d'un alliage de titane mélangé avantageusement avec un alliage à base de nickel

Et dans un mode préférentiel, le corps interne et/ou l'élément de fixation poreux sont constitués d'un matériau biocompatible contenant un matériau composite/binaire zircone-titane avec des concentrations de poudre zircone comprise entre 5 % et 25 %, qui est obtenu lors de la mise en forme de ce corps interne et/ou élément de fixation par fabrication additive par fusion sélective de couches de poudres, poudres avantageusement constituées d'un mélange de particules nanométriques de zircone (ou de tout autre céramiques) et de particules micrométriques de titane (ou de tout autre métal).

Une fois les formes du corps interne et de l'élément de fixation déterminées, la porosité de l'élément de fixation choisie, la cellule unitaire définissant cette porosité également choisie, les matériaux constitutifs de ceux-ci choisis, et le moyen de mobilité de l'élément de fixation déterminée, la réalisation par fabrication additive, et dans un mode préférentiel par la technologie SLM peut être lancée.

Le système d'implants dentaires à structures poreuses hétérogène est réalisé par fusion sélective de poudre métallique biocompatible avec un mode de réalisation particulier de la surface de l'implant.

Le système autobloquant de l'implant peut être réalisé par un moyen mécanique externe à l'implant ou interne et alors réalisé en même temps que l'implant et qui vient pousser la partie hélicoïdale vers le siège osseux de l'implant.

L'élément de fixation qui présente avantageusement un profil hélicoïdal afin d'être réparti sur l'ensemble de la surface externe de l'implant, et qui est rendu mobile entre les positions inactive rangée, et active déployée, peut être solidaire (a) ou non solidaire (b) du noyau central ou corps interne de l'implant dentaire :
(a) Solidaire : la partie hélicoïdale est maintenue par au moins quatre points de fixation solidaire du pilier central ou corps interne, à la pose de l'implant dentaire, un outil adapté vient chasser les points de fixation afin de libérer la partie hélicoïdale qui vient coller aux parois du creusé de l'implant. Dans ce cas, il peut être prévu que l'élément de fixation poreux soit fabriqué dans une forme pré-contrainte et fixé dans sa forme pré-contrainte au corps interne par les points de fixation, si bien que lors de la rupture des points de fixation, il puisse être libéré et se dilater naturellement vis à vis du corps interne pour adopter sa position active déployé
(b) Non solidaire : l'assemblage de la partie hélicoïdale sur le pilier central se fait par exemple par compression latérale par un outil adapté et à la pose de l'implant dentaire, l'ensemble est libéré.

D'autres moyens de blocage de l'élément de fixation dans le creusé formé dans l'os, peuvent être envisagés, citons à titre d'exemples la fixation thermique par dilatation thermique de l'élément de fixation lorsqu'inséré dans l'os du patient et soumis à sa température corporelle (on prévoit alors une dilatation thermique de l'élément de fixation supérieure à celle du corps interne de l'implant, d'au moins 20% par exemple) ou la fixation chimique en réaction à un additif ou au contact du milieu implantaire. Par exemple, l'élément de fixation peut être constitué d'un polymère réactif à l'humidité qui va gonfler lorsqu'exposé à l'humidité du corps du patient une fois inséré dans l'os.

Un autre mode de blocage peut être envisagé, avec un mélange stœchiométrique de Nickel/Titane formant le Nitinol, un alliage à mémoire de forme qui présente un coefficient de dilatation thermique et une élasticité supérieurs au titane. Dans ce cas, l'élément de fixation poreux pourra être constitué de Nitinol et le corps interne de titane.

### Premier mode de réalisation illustré

L'implant dentaire décrit dans la présente invention est composé d'un corps central qui représente le noyau de l'implant (1-1), la tête du corps central peut être arrondie, plate, creuse, ou tout forme particulière pour recevoir un pilier implantaire, dans un autre mode le noyau de l'implant peut être solidaire du pilier implantaire et réalisé en une seule pièce en fabrication additive avec des angles de courbure particuliers selon la morphologie.

Un revêtement à structures poreuses hétérogènes (1-2) de forme hélicoïdale pour certains implants mais ce revêtement peut être de différentes formes et géométries qui viennent en habillage d'une partie ou la totalité de l'implant dentaire.

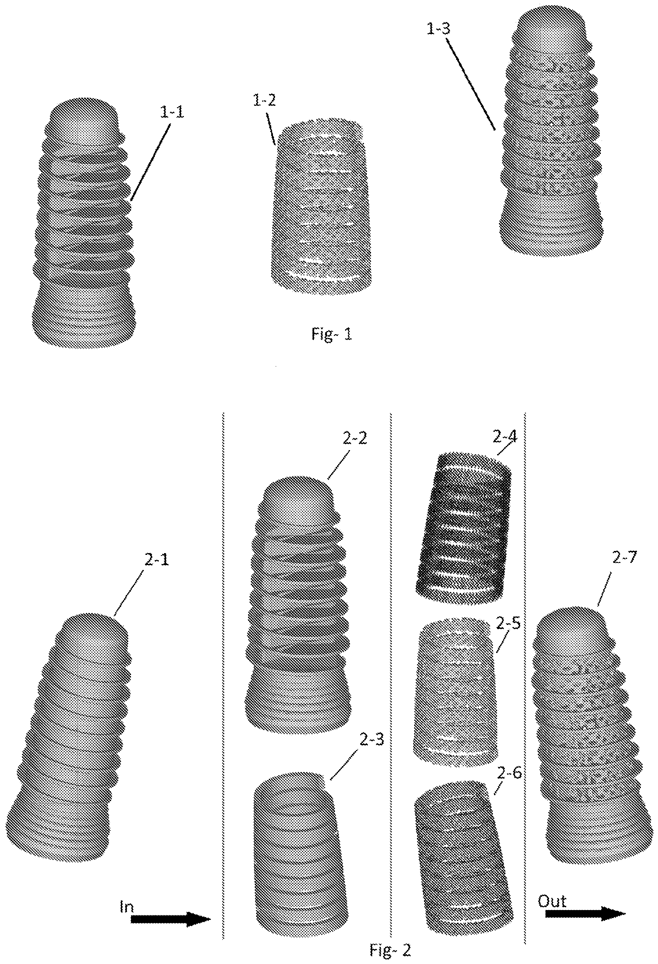

Dans l'exemple de la figure 1, le corps interne 1-1 présente une forme tronconique constituée d'une base évasée, d'une tête arrondie et d'une portion intermédiaire formée par une plaque verticale dont les bords latéraux sont traversés par une bande plate arrangée en forme de spirale, tel que mieux visible sur la figure 2 sous la référence 2-2. L'élément de fixation poreux est ici constitué d'une bande en forme de spirale, complémentaire de la spire formée par celle du corps interne et s'interposant entre les bords externes de deux spires consécutifs. Cette figure 1 représente une vue éclatée de l'implant selon l'invention sachant qu'il est préférentiellement obtenu en une pièce avec son corps interne et son élément de fixation poreux imbriqués l'un dans l'autre.

La figure 2 représente les étapes préliminaires à la réalisation proprement dite, étapes de définition de formes de bases à partir desquelles réaliser l'objet. Ainsi on défini numériquement l'enveloppe externe de l'implant à réaliser sans se soucier de la structure interne du corps telle que décrite ci-dessus, ni des pores de l'élément de fixation.

On définit ensuite la structure particulière du corps interne (2-2) et celle de l'élément de fixation, toujours sans les pores (2-3).

On définit ensuite différentes porosités pour l'élément de fixation (2-4, 2-5, 2-6).

Et après avoir choisi la porosité adaptée au cas particulier (en fonction des propriétés mécaniques envisagées avec une telle structure et une telle porosité), on définit un fichier réunissant le corps interne particulier avec sa structure complète, et l'élément de fixation avec la porosité désirée (2-7).

L'implant est ensuite réalisé par fabrication additive selon la technologie SLM déjà décrite, l'implant (2-1) est réalisé par un logiciel de conception en trois dimensions selon la morphologie du patient, la hauteur et la section de l'implant sont ainsi définis.

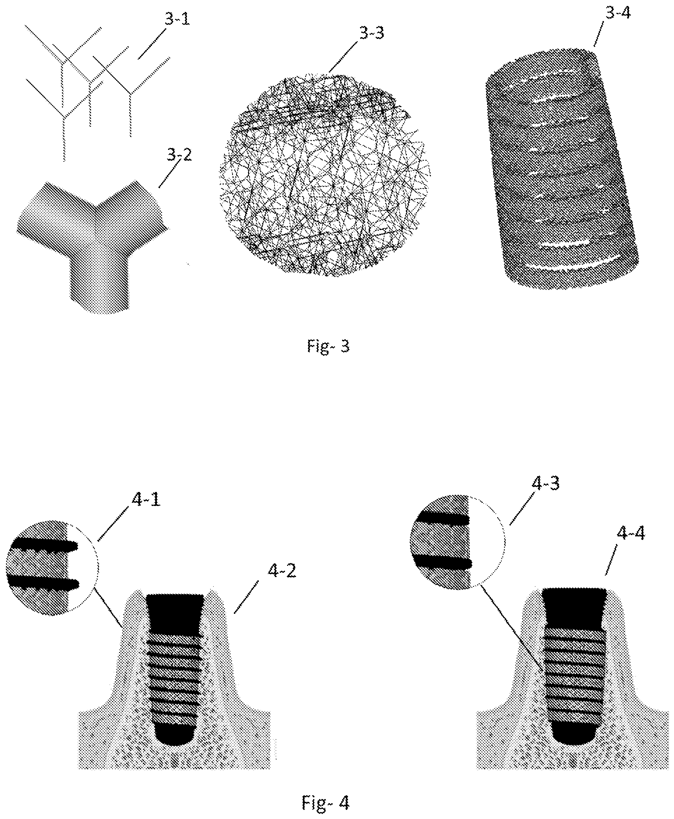

Dans cet exemple, l'élément de fixation est rendu mobile entre la position illustrée sur la figure 4 de gauche : position passive rangée, et la position active déployée (figure 4 droite) par le fait qu'il soit indépendant (non lié) au corps interne, et constitué d'un matériau présentant un coefficient de dilatation thermique supérieur de 15 à 40%, de préférence de 15 à 25% de celui du matériau constitutif du corps interne, à température corporelle (de l'ordre de 37°C). On pourrait prévoir également un élément de fixation réalisé solidaire du corps interne par des points de fixation sécables à partir d'un seuil de traction, seuil franchi lorsque l'élément de fixation se dilate thermiquement exposé à la température corporelle de l'utilisateur. On choisira alors le matériau en fonction de ce seuil.

Dans ce cas, l'élément de fixation se dilate de façon à ce que sa paroi externe vienne à fleur des crêtes de la bande à plat en spirale du corps et vient ainsi au contact sans espace de la cavité osseuse.

On peut prévoir que l'élément de fixation soit lié au corps par un pont de matière sécable (par exemple de moindre épaisseur) ou un pont chimique.

Ou que l'élément de fixation puisse être écarté radialement relativement au corps interne qui le contient.

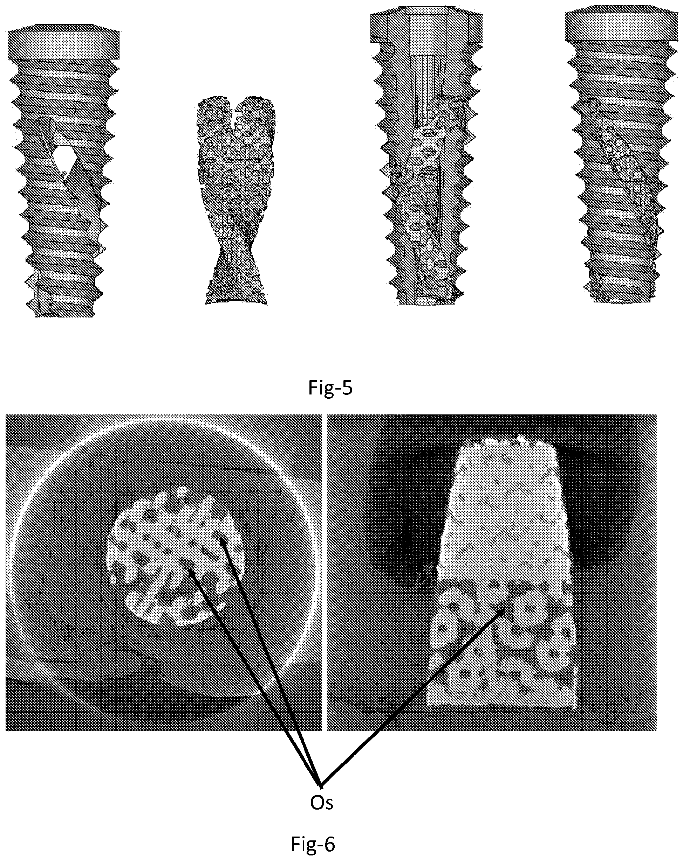

C'est le mode de réalisation représenté sur la figure 5.

La partie poreuse (2-3) est prédéfinie avec une épaisseur particulière comprise entre 1 et 2 mm du corps central de l'implant (2-2).

Plusieurs formes de porosité peuvent être sélectionné (2-4 ; 2-5 ; 2-6) avec la possibilité de paramétrer les zones de répartition de pores de différentes tailles.

La cellule de base ou cellule unitaire (3) est de forme géométrique en trois dimensions (x,y,z) ,la cellule unitaire est formée par au moins trois arêtes (3-1), avec un habillage de forme arrondi (3-2), cette forme n'est pas limitative, avec une ouverture d'angle d'au moins 10°, et La cellule unitaire peut être de forme régulière ou irrégulier en forme de pyramide, tétraèdre, cubique, octaèdre, icosaèdre, dodécaèdre et sans limitation de forme.

La répartition des cellules unitaires (3-3) peut être régulière ou irrégulière, avec des angles d'ouverture d'au moins 10°. Après habillage des cellules unitaires (3-4) la partie poreuse est constituée.

Un foret au diamètre de l'implant vient tarauder l'os, du fait de la faible densité et la porosité naturelle de l'os maxillaire et mandibulaire, le filetage est approximatif. A la mise en bouche de l'implant dentaire (4-2), de petites cavités apparaissent entre le corps de l'implant et l'os voir détail (4-1). A la libération de l'élément de fixation (4-4) toutes les petites cavités sont comblées, voir le détail (4-3).

Le flux d'exécution numérique des tâches est résumé dans la figure 2 :
Etape 1 : Le fichier numérique de l'implant dentaire est réalisé par un logiciel de conception en trois dimensions (2-1), la forme peut être standardisée selon un modèle pré défini ou personnalisable.
Etape 2 : délimitation de la partie de l'élément de fixation (2-3) et la partie solide représentant le corps de l'implant (2-2),
Etape 3 : génération d'un type de cellules unitaires ou une combinaison des dites cellules de base (2- 4 ; 2-5 ; 2-6). Lesquelles cellules se caractérisent par la géométrie ainsi réalisée présente l'avantage d'un contrôle de la porosité et sa densité.
Etape 4 : Réalisation de l'ensemble implant central+ élément de fixation (2-7) présentant une surface poreuse avantageusement hétérogène.

### Deuxième mode de réalisation

Dans cet exemple, le corps interne (5-1) présente une forme tronconique creuse définissant une épaisseur de paroi, pourvu d'un filetage externe 5-2 , et comprend deux gorges opposées 5-3 réalisées dans son épaisseur de paroi et suivant un profil hélicoïdal, l'élément de fixation 5-4 présentant la forme d'un ruban épais torsadé selon le profil hélicoïdal des deux gorges et d'une forme complémentaire à celles ci.

L'élément de fixation 3-4 comprend un évidemment longitudinal supérieur et central 5-5 de passage d'un dispositif d'écartement, tel que la vis de mise en place de l'implant.

Les deux premières vues de cette figure 5 représentent séparément le corps interne 5-1 et l'élément de fixation 5-4 pour une meilleure compréhension de leur structure, mais la forme réalisée en une seule pièce est bien celle représentée en vue avec arrachement et en vue normale sur les troisième et quatrième vue.

On peut voir sur la figure 6 qui représente une vue en coupe transversale et longitudinale d'un IRM réalisé sur un utilisateur portant un implant selon l'invention après 8 semaines de croissance osseuse, que l'os a pénétré profondément au sein de l'implant, et que la portion d'os ayant pénétré l'implant est de qualité équivalente à celle de l'os cortical entourant l''implant et donc, de bonne qualité.

### Exemple de paramètres de réalisation de l'ensemble Implant dentaire et élément de fixation sur une machine SLM 125 HL du fabricant SLM SOLUTIONS GMBH

| Type de paramètre | Implant dentaire | Elément de fixation |
|---|---|---|
| Type de poudre | Titane (Ti6Al4V) | Titane (Ti6Al4V) |
| Puissance du laser en zone pleine (w) | 400 | 300 |
| (w) Puissance basse du laser | 200 | 100 |
| Puissance du laser en zone poreuse (w) | | 100 |
| Exposition (ms) | 350 | 380 |
| Limite d'exposition (ms) | 300 | 350 |
| Distance de hachurage (µm) | 25 | 10 |

| Type de paramètre | Implant dentaire | Elément de fixation |
|---|---|---|
| Type de poudre | Titane/zir cone | Titane/nickel |
| Puissance du laser en zone pleine (w) | 400 | 200 |
| (w) Puissance basse du laser | 200 | 80 |
| Puissance du laser en zone poreuse (w) | | 80 |
| Exposition (ms) | 350 | 380 |
| Limite d'exposition (ms) | 300 | 350 |
| Distance de hachurage (µm) | 25 | 10 |

| Type de paramètre | Implant dentaire | Elément de fixation |
|---|---|---|
| Type de poudre | Titane/zir cone | Titane/zircone |
| Puissance du laser en zone pleine (w) | 400 | 400 |
| (w) Puissance basse du laser | 200 | 125 |
| Puissance du laser en zone poreuse (w) | | 125 |
| Exposition (ms) | 380 | 400 |
| Limite d'exposition (ms) | 280 | 320 |
| Distance de hachurage (µm) | 30 | 25 |

De manière préférentielle, il est rappelé que :
- l'ensemble suivant l'invention est réalisé par empilement de couches de poudres métalliques ou non métalliques, fusionnées sélectivement par concentration d'une source d'énergie.
- l'ensemble suivant l'invention est caractérisé en ce que ledit élément de fixation comporte une structure hétérogène présentant une porosité comprise entre 60% et 80%.
- l'ensemble suivant l'invention est caractérisé en ce que ledit élément de fixation est formé par un multiple d'une cellule unitaire, la dite cellule unitaire est formé par au moins trois arêtes et des angles d'ouvertures d'au moins 10°.
- l'ensemble suivant l'invention est caractérisé en ce que ledit implant dentaire possède soit une géométrie cylindro-conique, soit la forme morphologique de la dent.
- le matériau préféré selon l'invention est issu de la fusion d'un mélange homogène et stable de poudres de titane ou Ti6AIV4 microscopiques et de poudres de zircone yttriée nanoscopique dans lequel :
   * la teneur en titane ou Ti6AIV4est comprise entre 60 et 95,5%
   * avec une granulométrie comprise entre 5 et 10 microns
   * la teneur en zircone yttriée est comprise entre 13,5 et 30%
   * avec une granulométrie comprise entre 30 et 70 nm et une taille médiane comprise entre 65 et 85 nm et une taille minimale supérieure à 20 nm.

## Revendications

1. Ensemble formant un implant dentaire comprenant un corps interne (1-1, 5-1) de forme allongée ressemblant sensiblement à une racine de dent et un élément externe de fixation d'une structure osseuse à structure poreuse hétérogène (1-2, 5-4) apte à assurer la croissance osseuse, l'élément de fixation étant mobile entre une position passive rangée et une position active déployée, le corps interne et l'élément externe de fixation étant formés d'une seule pièce, **caractérisée en ce que** le corps interne (1-1, 5-1) de l'implant comprend un filetage externe présentant une hauteur de filetage H, l'élément de fixation est interposé entre les spires du filetage et se déployant entre la position passive rangée et la position active déployée d'une distance égale ou supérieure à la hauteur de filetage.

2. Ensemble selon la revendication précédente, dans lequel l'élément de fixation (1-2, 5-4) présente un coefficient de dilatation thermique supérieur à celui du corps de l'implant lui permettant un déploiement latéral supérieur de 15 à 40% vis à vis de celui du corps interne à température corporelle.

3. Ensemble selon la revendication précédente, dans lequel le matériau constitutif de l'élément de fixation (1-2, 5-4) présente un coefficient de dilatation thermique supérieur à celui du corps de l'implant lui permettant un déploiement latéral supérieur de 15 à 40% vis à vis de celui du corps interne à température corporelle.

4. Ensemble selon l'une des revendications précédentes, comprenant un dispositif amovible d'écartement de l'élément de fixation qui est inséré à l'intérieur du corps interne et qui commande le déplacement de l'élément de fixation (1-2, 5-4) de la position passive rangée à la position active déployée.

5. Ensemble selon la revendication précédente, dans lequel le dispositif amovible d'écartement est une vis de mise en place de l'implant.

6. Ensemble selon l'une des revendications précédentes, dans lequel l'élément de fixation (1-2, 5-4) est relié au corps interne par une liaison sécable.

7. Ensemble selon l'une des revendications 1 à 5, dans lequel l'élément de fixation (1-2, 5-4) et le corps interne ne présentent aucune liaison.

8. Ensemble selon l'une des revendications précédentes, dans lequel l'élément de fixation et/ou le corps interne sont constitués d'un matériau composite formé d'une fusion de microparticules d'un premier matériau à température de fusion donné, et de nanoparticules d'un deuxième matériau à température de fusion plus élevé, formant un réseau de zones dendritiques du premier matériau à l'échelle du micron entremêlé de zones filamenteuses nanométriques du deuxième matériau.

9. Ensemble selon la revendication précédente, dans lequel le premier matériau est un métal.

10. Ensemble selon la revendication précédente, dans lequel le premier matériau est ou comprend du titane.

11. Ensemble selon la revendication précédente, dans lequel le deuxième matériau est une céramique.

12. Ensemble selon la revendication précédente, dans lequel le deuxième matériau est une zircone.

13. Ensemble selon la revendication précédente, dans lequel le deuxième matériau est une zircone yttriée.

14. Ensemble selon l'une des revendications précédentes, dans lequel le corps interne présente une forme tronconique creuse définissant une épaisseur de paroi, pourvu d'un filetage externe, et comprend deux gorges opposées réalisées dans son épaisseur de paroi et suivant un profil hélicoïdal, l'élément de fixation présentant la forme d'un ruban épais torsadé selon le profil hélicoïdal des deux gorges et d'une forme complémentaire à celles ci.

15. Ensemble selon la revendication précédente, dans lequel l'élément de fixation et/ou le corps interne comprend un évidemment longitudinal central de passage d'un dispositif d'écartement de l'élément de fixation.

16. Procédé de fabrication d'un ensemble formant implant dentaire selon l'une des revendications précédentes, par empilement de couches de poudres métalliques et/ou non métalliques, fusionnées sélectivement par concentration d'une source d'énergie comprenant une étape de préparation de la mobilité de l'élément de fixation par rapport au corps interne.

17. Procédé selon la revendication précédente, dans lequel l'étape de préparation de la mobilité de l'élément de fixation comprend une étape de formation d'un pont de matière sécable entre une partie de l'ensemble formant le corps interne et une partie de l'ensemble formant l'élément de fixation lors de la fabrication par empilement de couches.

18. Procédé selon la revendication 16, dans lequel l'étape de préparation de la mobilité de l'élément de fixation comprend une étape de constitution de la partie de l'ensemble formant l'élément de fixation avec un matériau présentant un coefficient de dilatation thermique supérieur à celui du corps de l'implant lui permettant un déploiement latéral supérieur de 15 à 40% vis à vis de celui du corps interne à température corporelle.

19. Procédé selon l'une des revendications 16 à 18, dans lequel 'étape de préparation de la mobilité de l'élément de fixation comprend une étape de formation de la partie de l'ensemble formant le corps interne et de la partie de l'ensemble formant l'élément de fixation sans pont de matière entre elles, la partie de l'ensemble formant l'élément de fixation étant fabriquée avec un évidemment interne lui permettant d'être déployable radialement sous l'effet d'une dilatation thermique ou l'action d'un outil, indépendamment de la partie de l'ensemble formant le corps interne.

## Patentansprüche

1. Anordnung, die ein Zahnimplantat bildet, das einen inneren Körper (1-1, 5-1) mit länglicher Form, der im Wesentlichen einer Zahnwurzel ähnelt, und ein äußeres Element zum Fixieren einer Knochenstruktur mit einer heterogenen porösen Struktur (1-2, 5-4) umfasst, die geeignet ist, das Knochenwachstum sicherzustellen, wobei das Fixierelement zwischen einer passiven nicht aufgeweiteten Position und einer aktiven aufgeweiteten Position beweglich ist, wobei der innere Körper und das äußere Fixierelement in einem Stück ausgebildet sind, **dadurch gekennzeichnet, dass** der innere Körper (1-1, 5-1) des Implantats ein Außengewinde mit einer Gewindehöhe H umfasst, das Fixierelement zwischen den Windungen des Gewindes angeordnet ist und sich zwischen der passiven nicht aufgeweiteten Position und der aktiven aufgeweiteten Position über eine Strecke, die gleich oder größer als die Gewindehöhe ist, aufweitet.

2. Anordnung nach dem vorhergehenden Anspruch, wobei das Fixierelement (1-2, 5-4) einen Wärmeausdehnungskoeffizienten aufweist, der größer als der des Körpers des Implantats ist, was eine größere seitliche Aufweitung von 15 bis 40 % gegenüber der des inneren Körpers bei Körpertemperatur ermöglicht.

3. Anordnung nach dem vorhergehenden Anspruch, wobei das Material, aus dem das Fixierelement (1-2, 5-4) besteht, einen Wärmeausdehnungskoeffizienten aufweist, der größer als der des Körpers des Implantats ist, was eine größere seitliche Aufweitung von 15 bis 40 % gegenüber der des inneren Körpers bei Körpertemperatur ermöglicht.

4. Anordnung nach einem der vorhergehenden Ansprüche, umfassend eine entnehmbare Abstandhaltervorrichtung des Fixierelements, die in den inneren Körper eingeführt ist und die Bewegung des Fixierelements (1-2, 5-4) von der passiven nicht aufgeweiteten Position zur aktiven aufgeweiteten Position auslöst.

5. Anordnung nach dem vorhergehenden Anspruch, wobei die entnehmbare Abstandhaltervorrichtung eine Schraube zum Einsetzen des Implantats ist.

6. Anordnung nach einem der vorhergehenden Ansprüche, wobei das Fixierelement (1-2, 5-4) durch eine trennbare Verbindung mit dem inneren Körper verbunden ist.

7. Anordnung nach einem der Ansprüche 1 bis 5, wobei das Fixierelement (1-2, 5-4) und der inneren Körper keine Verbindung aufweisen.

8. Anordnung nach einem der vorhergehenden Ansprüche, wobei das Fixierelement und/oder der innere Körper aus einem Verbundmaterial bestehen, das aus einer Verschmelzung von Mikropartikeln eines ersten Materials mit einer gegebenen Schmelztemperatur und von Nanopartikeln eines zweiten Materials mit einer höheren Schmelztemperatur gebildet wird, wodurch ein Netzwerk von dendritischen Zonen aus dem ersten Material im Mikrometerbereich, die von nanometrischen filamentösen Zonen aus dem zweiten Material durchsetzt sind, gebildet wird.

9. Anordnung nach dem vorhergehenden Anspruch, wobei das erste Material ein Metall ist.

10. Anordnung nach dem vorhergehenden Anspruch, wobei das erste Material Titan ist oder umfasst.

11. Anordnung nach dem vorhergehenden Anspruch, wobei das zweite Material ein Keramikmaterial ist.

12. Anordnung nach dem vorhergehenden Anspruch, wobei das zweite Material ein Zirkonoxid ist.

13. Anordnung nach dem vorhergehenden Anspruch, wobei das zweite Material ein Yttriumoxid-stabilisiertes Zirkonoxid ist.

14. Anordnung nach einem der vorhergehenden Ansprüche, wobei der innere Körper eine hohle, kegelstumpfförmige Form aufweist, die eine Wandstärke definiert, mit einem Außengewinde versehen ist und zwei sich gegenüberliegende Rillen aufweist, die in ihrer Wandstärke hergestellt sind und einem Schraubenprofil folgen, wobei das Fixierelement die Form eines dicken Bandes aufweist, das entlang des spiralförmigen Profils der beiden Rillen verdrillt ist und eine dazu komplementäre Form aufweist.

15. Anordnung nach dem vorhergehenden Anspruch, wobei das Fixierelement und/oder der innere Körper eine zentrale Längsaussparung für den Durchgang einer Abstandhaltervorrichtung des Fixierelements umfasst.

16. Verfahren zur Herstellung einer Anordnung, wobei ein Zahnimplantat nach einem der vorhergehenden Ansprüche durch Stapeln von Schichten aus metallischen und/oder nichtmetallischen Pulvern gebildet wird, die selektiv durch Konzentration einer Energiequelle verschmolzen sind, umfassend einen Schritt der Herstellung der Beweglichkeit des Fixierelements relativ zu dem inneren Körper.

17. Verfahren nach dem vorhergehenden Anspruch, wobei der Schritt der Herstellung der Beweglichkeit des Fixierelements einen Schritt der Bildung einer Brücke aus trennbarem Material zwischen einem Teil der Anordnung, der den inneren Körper bildet, und einem Teil der Anordnung, der das Fixierelement bildet, während der Herstellung durch Stapeln von Schichten umfasst.

18. Verfahren nach Anspruch 16, wobei der Schritt zur Herstellung der Beweglichkeit des Fixierelements einen Schritt zur Bildung des Teils der Anordnung, der das Fixierelement bildet, mit einem Material mit einem höheren Wärmeausdehnungskoeffizienten gegenüber dem des Körpers des Implantats umfasst, wodurch diesem eine seitliche Aufweitung, die 15 bis 40 % größer ist als die des inneren Körpers bei Körpertemperatur, ermöglicht wird.

19. Verfahren nach einem der Ansprüche 16 bis 18, wobei der Schritt der Herstellung der Beweglichkeit des Fixierelements einen Schritt der Bildung des Teils der Anordnung, der den inneren Körper bildet, und des Teils der Anordnung, der das Fixierelement bildet, ohne eine Materialbrücke dazwischen umfasst, wobei der Teil der Anordnung, der das Fixierelement bildet, mit einer inneren Aussparung hergestellt wird, die es ermöglicht, dass es unabhängig von dem Teil der Anordnung, der den inneren Körper bildet, unter dem Einfluss der Wärmeausdehnung oder der Wirkung eines Werkzeugs radial aufweitbar ist.

## Claims

1. Assembly forming a dental implant comprising an elongate inner body (1-1, 5-1) substantially resembling a tooth root and an outer element (1-2, 5-4) for fastening a bone structure that has a heterogeneous porous structure and is capable of providing bone growth, the fastening element being movable between a passive retracted position and an active extended position, the inner body and the outer fastening element being formed in one piece, **characterized in that** the inner body (1-1, 5-1) of the implant comprises an outer thread having a thread height H, and the fastening element is interposed between the turns of the thread and extends, between the passive retracted position and the active extended position, a distance equal to or greater than the thread height.

2. Assembly according to the preceding claim, wherein the fastening element (1-2, 5-4) has a coefficient of thermal expansion greater than that of the body of the implant, allowing said element a lateral extension that is 15 to 40% greater than that of the inner body at body temperature.

3. Assembly according to the preceding claim, wherein the constituent material of the fastening element (1-2, 5-4) has a coefficient of thermal expansion greater than that of the body of the implant, allowing said material a lateral extension that is 15 to 40% greater that of the inner body at body temperature.

4. Assembly according to any of the preceding claims, comprising a removable device for spacing the fastening element, which device is inserted inside the inner body and controls the movement of the fastening element (1-2, 5-4) from the passive retracted position to the active extended position.

5. Assembly according to the preceding claim, wherein the removable spacer device is a screw for positioning the implant.

6. Assembly according to any of the preceding claims, wherein the fastening element (1-2, 5-4) is connected to the inner body by a breakable connection.

7. Assembly according to any of claims 1 to 5, wherein the fastening element (1-2, 5-4) and the inner body have no connection.

8. Assembly according to any of the preceding claims, wherein the fastening element and/or the inner body are made of a composite material formed by melting microparticles of a first material having a given melting point, and nanoparticles of a second material having a higher melting point, forming a network of dendritic regions of the first material at the micron scale interspersed with nanometric filamentous regions of the second material.

9. Assembly according to the preceding claim, wherein the first material is a metal.

10. Assembly according to the preceding claim, wherein the first material is or comprises titanium.

11. Assembly according to the preceding claim, wherein the second material is a ceramic material.

12. Assembly according to the preceding claim, wherein the second material is zirconia.

13. Assembly according to the preceding claim, wherein the second material is yttriated zirconia.

14. Assembly according to any of the preceding claims, wherein the inner body has a hollow frustoconical shape defining a wall thickness, provided with an outer thread, and comprises two opposite grooves made in its wall thickness and following a helical profile, the fastening element having the form of a thick ribbon twisted according to the helical profile of the two grooves and having a shape complementary to said grooves.

15. Assembly according to the preceding claim, wherein the fastening element and/or the inner body comprises a central longitudinal recess for the passage of a device for spacing the fastening element.

16. Method for producing a dental implant assembly according to any of the preceding claims by stacking layers of metal and/or non-metal powders, the layers being selectively melted by focusing an energy source, the method comprising a step of preparing the mobility of the fastening element relative to the inner body.

17. Method according to the preceding claim, wherein the step of preparing the mobility of the fastening element comprises a step of forming a bridge of breakable material between a part of the assembly forming the inner body and a part of the assembly forming the fastening element during the production by stacking layers.

18. Method according to claim 16, wherein the step of preparing the mobility of the fastening element comprises a step of making the part of the assembly forming the fastening element using a material having a coefficient of thermal expansion that is higher than that of the body of the implant, allowing said part a lateral extension that is 15 to 40% greater than that of the inner body at body temperature.

19. Method according to any of claims 16 to 18, wherein the step of preparing the mobility of the fastening element comprises a step of forming the part of the assembly forming the inner body and the part of the assembly forming the fastening element without a bridge of material therebetween, the part of the assembly forming the fastening element being produced having an inner recess, allowing said part to be radially extendable under thermal expansion or when acted upon by a tool, independently of the part of the assembly forming the inner body.
